# EUROPEAN PATENT SPECIFICATION

(11) **EP 3 887 331 B1**
(45) Date of publication and mention of the grant of the patent: **14.08.2024**
(21) Application number: 19817892.3
(22) Date of filing: 18.11.2019
(51) Int. Cl.: C03C 3/097, C03C 4/00, C03C 12/00, C03C 13/00

(54) **BIOACTIVE SILICATE GLASSES**
BIOAKTIVE SILIKATGLÄSER
VERRES BIOACTIFS AU SILICATE

(30) Priority: 26.11.2018 US 201862771341 P; 25.01.2019 US 201962796859 P
(43) Date of publication of application: 06.10.2021
(73) Proprietor: Corning Incorporated, Corning, New York 14831 (US)
(72) Inventor: FU, Qiang, Painted Post, New York 14870 (US)
(74) Representative: Elkington and Fife LLP
(86) International application number: PCT/US2019/061988
(87) International publication number: WO 2020/112404

(56) References cited:
- WO-A1-96/21628
- US-A- 4 171 544
- US-A1- 2005 064 193
- US-B2- 7 074 730
- US-B2- 7 141 520

## Description

### BACKGROUND

### 1. Field

The disclosure relates to bioactive glasses for use in biomedical applications. In particular, the glasses described herein are silicate glasses that show fast filling rates of dentin tubules and have advantageous release rates of metal ions, which provide advantages in antibacterial applications and wound healing.

### 2. Technical Background

Bioactive glasses are a group of glass and glass ceramic materials that have shown biocompatibility or bioactivity, which has allowed them to be incorporated into human or animal physiology. A number of these materials exist on the market already, such as Bioglass 8625, a soda-lime glass used for encapsulation of implanted devices, and Bioglass 45S5, a bioactive glass composition used in bone repair. However, there continues to be an unmet need for solutions to biomedical problems that novel biocompatible inorganic compositions may help resolve.

This disclosure presents improved biocompatible inorganic compositions and methods of manufacturing thereof for biomedical applications. US 2005/064193 A1, US 7141520 B2, US 7074730 B2 are directed, *inter alia,* to powdered glass with antimicrobial properties. WO 96/21628 A1 relates to bioactive glass fibers for use in the medical field, in particular for tissue bonding purposes. US 4171544 A relates to glass material deposited on a surface for bonding to the bone.

### SUMMARY

The invention relates to an antibacterial composition, comprising: a silicate-based glass material being a fiber and having a composition of: 60-70 wt.% SiO₂, 0-10 wt.% B₂O₃, 3-18 wt.% P₂O₅, 0-10 wt.% Al₂O₃, 0-5 wt.% Li₂O, 20-30 wt.% Na₂O, 0-15 wt.% K₂O, 0-10 wt.% MgO, 1-15 wt.% CaO, 2-20 wt.% MO, and 20-35 wt.% R₂O, wherein MO is the sum of MgO, CaO, SrO, and BaO, wherein R₂O is the sum of Na₂O, K₂O, Li₂O, and Rb₂O, and wherein the silicate-based glass material is configured to achieve a 6-log kill rate of at least one of *E. coli*, *P. gingivalis*, or *S. mutans* bacteria.

### BRIEF DESCRIPTION OF THE DRAWINGS

The disclosure will become more fully understood from the following detailed description, taken in conjunction with the accompanying figures, in which:
FIG. 1 illustrates a particle size distribution of Example Composition 5 after air jet milling.
FIG. 2 illustrates pH changes of a culture medium as a function of time after soaking with Example Composition 5.
FIG. 3 illustrates a bar graph plot of kill rate of *E. coli* as a function of pH change of a culture medium.
FIG. 4 illustrates a bar graph plot of the kill rate of *P. Gingivalis* after 1, 2 and 7 days of incubation with Example Composition 5.
FIG. 5 illustrates a bar graph plot of the kill rate of *S. Mutans* after 1, 2 and 7 days of incubation with Example Composition 5.

### DETAILED DESCRIPTION

In the following description, whenever a group is described as comprising at least one of a group of elements and combinations thereof, it is understood that the group may comprise, consist essentially of, or consist of any number of those elements recited, either individually or in combination with each other. Similarly, whenever a group is described as consisting of at least one of a group of elements or combinations thereof, it is understood that the group may consist of any number of those elements recited, either individually or in combination with each other. Unless otherwise specified, a range of values, when recited, includes both the upper and lower limits of the range as well as any ranges therebetween. As used herein, the indefinite articles "a," "an," and the corresponding definite article "the" mean "at least one" or "one or more," unless otherwise specified. It also is understood that the various features disclosed in the specification and the drawings can be used in any and all combinations.

Where a range of numerical values is recited herein, comprising upper and lower values, unless otherwise stated in specific circumstances, the range is intended to include the endpoints thereof, and all integers and fractions within the range. It is not intended that the scope of the claims be limited to the specific values recited when defining a range. Further, when an amount, concentration, or other value or parameter is given as a range, one or more preferred ranges or a list of upper preferable values and lower preferable values, this is to be understood as specifically disclosing all ranges formed from any pair of any upper range limit or preferred value and any lower range limit or preferred value, regardless of whether such pairs are separately disclosed. Finally, when the term "about" is used in describing a value or an end-point of a range, the disclosure should be understood to include the specific value or end-point referred to. When a numerical value or end-point of a range does not recite "about," the numerical value or end-point of a range is intended to include two embodiments: one modified by "about," and one not modified by "about."

As used herein, the term "about" means that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art. It is noted that the terms "substantially" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue. Thus, a glass that is "free" or "essentially free" of Al₂O₃ is one in which Al₂O₃ is not actively added or batched into the glass, but may be present in very small amounts as a contaminant (e.g., 500, 400, 300, 200, or 100 parts per million (ppm) or less or).

Herein, glass compositions are expressed in terms of wt% amounts of particular components included therein on an oxide bases unless otherwise indicated. Any component having more than one oxidation state may be present in a glass composition in any oxidation state. However, concentrations of such component are expressed in terms of the oxide in which such component is at its lowest oxidation state unless otherwise indicated.

Unless otherwise specified, all compositions are expressed in terms of weight percent (wt%). Coefficients of thermal expansion (CTE) are expressed in terms of 10⁻⁷/°C, unless otherwise specified. The CTE can be determined, for example, using the procedure described in ASTM E228 "Standard Test Method for Linear Thermal Expansion of Solid Materials with a Push-Rod Dilatometer" or ISO 7991:1987 "Glass -- Determination of coefficient of mean linear thermal expansion." The density in terms of grams/cm³ was measured via the Archimedes method (ASTM C693). Young's modulus, shear modulus, and Poisson's Ratio were measured via the ASTM C623 standard.

### Glass Compositions

Bioactive glasses are a group of glass and glass ceramic materials that have shown biocompatibility or bioactivity, which has allowed them to be incorporated into human or animal physiology. The biocompatibility and *in vivo* properties of the glass are influenced by the glass composition. In the glass compositions described herein, SiO₂ serves as the primary glass-forming oxide in combination with the bioactive oxides of calcium and phosphorous.

The glass comprises a combination of SiO₂, Na₂O, P₂O₅, and CaO. In some examples, the glass further comprises Al₂O₃, B₂O₃, K₂O, and/or Li₂O. The glass comprises a glass composition comprising, in wt.%: 60-70 SiO₂, 0-10 B₂O₃, 3-18 P₂O₅, 0-10 Al₂O₃, 0-5 Li₂O, 20-30 Na₂O, 0-15 K₂O, 0-10 MgO, 1-15 CaO, 2-20 MO, and 20-35 R₂O, wherein MO is the sum of MgO, CaO, SrO, and BaO, R₂O is the sum of Na₂O, K₂O, Li₂O, and Rb₂O. In some examples, the glass composition comprises 5-15 wt.% P₂O₅. In some examples, the glass composition additionally comprises >0-8 wt.% ZnO. The silicate glasses disclosed herein are particularly suitable for biomedical or bioactive applications.

Silicon dioxide (SiO₂), which is the major oxide component of the embodied glasses, may be included to provide high temperature stability and chemical durability. The glass comprises 60-70 wt.% SiO₂. In some examples, the glass can comprise 60-65 wt.%, or 65-70 wt.% SiO₂. In some examples, the glass comprises 60, 61, 62, 63, 64, 65, 66, 67, 68, 69, 70 wt.% SiO₂.

The glass comprises 0-10 wt.% B₂O₃. In some examples, the glass can comprise >0-10 wt.% B₂O₃. In some examples, the glass can comprise 0-5 wt.% B₂O₃. In some examples, the glass can comprise from >0-10 wt.%, 2-10 wt.%, 5-10 wt.%, 0-8 wt.%, >0-8 wt.%, 2-8 wt.%, 5-8 wt.%, 0-5 wt.%, >0-5 wt.%, or 2-5 wt.% B₂O₃. In some examples, the glass can comprise 0, >0, 1, 2, 3, 4, 5, 6, 7, 8, 9, or 10 wt.% B₂O₃.

Alumina (Al₂O₃) may influence the structure of the glass and, additionally, lower the liquidus temperature and coefficient of thermal expansion, or enhance the strain point. In addition to its role as a network former, Al₂O₃ (and ZrO₂) help improve the chemical durability in silicate glass while having no toxicity concerns. The glass comprises 0-10 wt.% Al₂O₃. In some examples, the glass can comprise from 0 to 8 wt.%, 0 to 6 wt.%, 0 to 4 wt.%, 0 to 2 wt.%, >0 to 10 wt.%, >0 to 8 wt.%, >0 to 6 wt.%, >0 to 4 wt.%, >0 to 2 wt.%, 1 to 10 wt.%, 1 to 8 wt.%, 1 to 6 wt.%, 1 to 4 wt.%, 1 to 2 wt.%, 3 to 8 wt.%, 3 to 6 wt.%, 3 to 10 wt.%, 5 to 8 wt.%, 5 to 10 wt.%, 7 to 10 wt.%, or 8 to 10 wt.% Al₂O₃. In some examples, the glass can comprise 0, >0, 1, 2, 3, 4, 5, 6, 7, 8, 9, or 10 wt.% Al₂O₃.

Phosphorus pentoxide (P₂O₅) also serves as a network former. Furthermore, the liberation of phosphate ions to the surface of bioactive glasses contributes to the formation of apatite. The inclusion of phosphate ions in the bioactive glass increases apatite formation rate and the binding capacity of the bone tissue. In addition, P₂O₅ increases the viscosity of the glass, which in turn expands the range of operating temperatures, and is therefore an advantage to the manufacture and formation of the glass. Tthe glass comprises 3-18 wt.% P₂O₅. In some examples, the glass can comprise 3-15 wt.%, 3-12 wt.%, 3-10 wt.%, 3-8 wt.%, 3-5 wt.%, 5-18 wt.%, 5-15 wt.%, 5-12 wt.%, 5-10 wt.%, 5-8 wt.%, 8-18 wt.%, 8-15 wt.%, 8-12 wt.%, 8-10 wt.%, 10-18 wt.%, 10-15 wt.%, 10-12 wt.%, 12-18 wt.%, or 12-15 wt.% P₂O₅. In some examples, the glass can comprise about 3, 4, 5, 6, 7, 8, 9, 10, 11, 12, 13, 14, 15, 16, 17, or 18 wt.% P₂O₅.

Alkali oxides (Li₂O, Na₂O, K₂O, Rb₂O, or Cs₂O) serve as aids in achieving low melting temperature and low liquidus temperatures. Meanwhile, the addition of alkali oxides can improve bioactivity. Further, Na₂O and K₂O may influence the coefficient of thermal expansion, especially at low temperatures. The glass comprises from 20-30 wt.% Na₂O. In some examples, the glass can comprise 20-25 wt.% Na₂O. In some examples, the glass can comprise from 20-28 wt.%, 20-25 wt.%, or 25-30 wt.% Na₂O. In some examples, the glass can comprise 20, 21, 22, 23, 24, 25, 26, 27, 28, 29, or 30 wt.% Na₂O.

The glass comprises from 0-15 wt.% K₂O. In some examples, the glass can comprise 0-10 wt.% K₂O. In some examples, the glass can comprise >0-5 wt.% K₂O. In some examples, the glass can comprise >0-15 wt.%, 2-15 wt.%, 5-15 wt.%, 8-15 wt.%, 10-15 wt.%, 0-10 wt.%, >0-10 wt.%, 2-10 wt.%, 5-10 wt.%, 0-8 wt.%, >0-8 wt.%, 2-8 wt.%, 5-8 wt.%, 0-5 wt.%, >0-5 wt.%, or 2-5 wt.% K₂O. In some examples, the glass can comprise 0, >0, 1, 2, 3, 4, 5, 6, 7, 8, 9, 10, 11, 12, 13, 14, or 15 wt.% K₂O.

In some examples, the total amount of Na₂O and K₂O is important to the properties of the glass. In such examples, the glass can comprise a total of 20-35 wt.% Na₂O and K₂O combined.

The glass comprises from 0-5 wt.% Li₂O. In some examples, the glass can comprise from >0-5 wt.% Li₂O. In some examples, the glass can comprise from about >0-3.5 wt.% Li₂O. In some examples, the glass can comprise from 1-4 wt.% Li₂O. In some examples, the glass can comprise from 0-4 wt.%, 0-3 wt.%, 0-2 wt.%, >0 to 5 wt.%, >0 to 4 wt.%, >0 to 3 wt.%, >0 to 2 wt.%, 1 to 5 wt.%, 1 to 4 wt.%, or 1 to 3 wt.% Li₂O. In some examples, the glass can comprise about 0, >0, 1, 2, 3, 4, or 5 wt.% Li₂O.

In some examples, the total amount of the alkalis Li₂O, Na₂O, and K₂O (R₂O) is important to the glass properties. In some examples, the glass can comprise 12-35 wt.% R₂O, wherein R₂O is the sum or Li₂O, Na₂O, and K₂O. In some examples, the glass can comprise 20-35 wt.% R₂O. In some examples, the glass can comprise from 20-30 wt.%, 20-28 wt.%, 20-25 wt.%, 22-35 wt.%, 22-30 wt.%, 22-28 wt.%, 25-35 wt.%, or 25-30 wt.% R₂O. In some examples, the glass can comprise 20, 21, 22, 23, 24, 25, 26, 27, 28, 29, 30, 31, 32, 33, 34, or 35 wt.% R₂O.

Divalent cation oxides (such as alkaline earth oxides) also improve the melting behavior and the bioactivity of the glass. Particularly, CaO is found to be able to react with P₂O₅ to form apatite when immersed in a simulated body fluid (SBF) or in vivo. The release of Ca²⁺ ions from the surface of the glass contributes to the formation of a layer rich in calcium phosphate.

The glass comprises 1-15 wt.% CaO. In some examples, the glass can comprise 1-10 wt.% CaO. In some examples, the glass can comprise from 1-12 wt.%, 1-10 wt.%, 1-8 wt.%, 3-15 wt.%, 3-12 wt.%, 3-10 wt.%, 3-8 wt.%, 5-15 wt.%, 5-12 wt.%, 3-10 wt.%, 5-8 wt.%, 8-15 wt.%, 8-12 wt.%, 8-10 wt.%, or 10-15 wt.% CaO. In some examples, the glass can comprise 1, 2, 3, 4, 5, 6, 7, 8, 9, 10, 11, 12, 13, 14, or 15 wt.% CaO.

The combination of P₂O₅ and CaO may provide advantageous compositions for bioactive glasses. In some examples, the glass compositions comprise P₂O₅ and CaO with the sum of P₂O₅ and CaO being from 6-25 wt.%, 8-20 wt.% or 8-15 wt.%.

The glass comprises 0-10 wt.% MgO. In some examples, the glass can comprise from 0 to 5 wt.% MgO. In some examples, the glass can comprise from >0 to 10 wt.%, 3 to 10 wt.%, or 3 to 8 wt.% MgO. In some examples, the glass can comprise from 0 to 8 wt.%, 0 to 6 wt.%, 0 to 4 wt.%, 0 to 2 wt.%, >0 to 10 wt.%, >0 to 8 wt.%, >0 to 6 wt.%, >0 to 4 wt.%, >0 to 2 wt.%,1 to 10 wt.%, 1 to 8 wt.%, 1 to 6 wt.%, 1 to 4 wt.%, 1 to 2 wt.%, 3 to 8 wt.%, 3 to 6 wt.%, 3 to 10 wt.%, 5 to 8 wt.%, 5 to 10 wt.%, 7 to 10 wt.%, or 8 to 10 wt.% MgO. In some examples, the glass can comprise about 0, >0, 1, 2, 3, 4, 5, 6, 7, 8, 9, or 10 wt.% MgO.

Strontium oxide (SrO) may be present in some embodiments and in such examples, the glass can comprise from 0 to 10 wt.% SrO. In some examples, the glass can comprise from >0 to 10 wt.% SrO. In some examples, the glass can comprise from 3 to 10 wt.%, 5 to 10 wt.%, 5 to 8 wt.% SrO. In some examples, the glass can comprise from 0 to 10 wt.%, 0 to 8 wt.%, 0 to 6 wt.%, 0 to 4 wt.%, 0 to 2 wt.%, >0 to 10 wt.%, >0 to 8 wt.%, >0 to 6 wt.%, >0 to 4 wt.%, >0 to 2 wt.%, 1 to 10 wt.%, 1 to 8 wt.%, 1 to 6 wt.%, 1 to 4 wt.%, 1 to 2 wt.%, 3 to 8 wt.%, 3 to 6 wt.%, 3 to 10 wt.%, 5 to 8 wt.%, 5 to 10 wt.%, 7 to 10 wt.%, or 8 to 10 wt.% SrO. In some examples, the glass can comprise about >0, 1, 2, 3, 4, 5, 6, 7, 8, 9, or 10 wt.% SrO.

Barium oxide (BaO) may be present in some examples and in such examples, the glass can comprise from 0 to 15 wt.% BaO. In some examples, the glass can comprise from 0 to 10 wt.%, >0 to 5 wt.%, 6 to 13 wt.%, 5 to 15 wt.%, 7 to 13 wt.%, 7 to 11 wt.%, 8 to 12 wt.% BaO. In some examples, the glass can comprise from 0 to 15 wt.%, 0 to 13 wt.%, 0 to 11 wt.%, 0 to 9 wt.%, 0 to 7 wt.%, 0 to 5 wt.%, >0 to 15 wt.%, >0 to 13 wt.%, >0 to 11 wt.%, >0 to 9 wt.%, >0 to 7 wt.%, >0 to 5 wt.%, 1 to 15 wt.%, 1 to 13 wt.%, 1 to 11 wt.%, 1 to 9 wt.%, 1 to 7 wt.%, 1 to 5 wt.%, 3 to 15 wt.%, 3 to 13 wt.%, 3 to 11 wt.%, 3 to 9 wt.%, 3 to 7 wt.%, 3 to 5 wt.%, 5 to 15 wt.%, 5 to 13 wt.%, 5 to 11 wt.%, 5 to 9 wt.%, 5 to 7 wt.%, 7 to 15 wt.%, 7 to 13 wt.%, 7 to 11 wt.%, 7 to 9 wt.%, 9 to 15 wt.%, 9 to 13 wt.%, 9 to 11 wt.%, 11 to 15 wt.%, or 11 to 13 wt.% BaO. In some examples, the glass can comprise about 0, >0, 1, 2, 3, 4, 5, 6, 7, 8, 9, 10, 11, 12, 13, 14, or 15 wt.% BaO.

Alkaline earth oxides may improve other desirable properties in the materials, including influencing the Young's modulus and the coefficient of thermal expansion. The glass comprises from 2-20 wt.% MO (2 wt.% ≤ MO ≤ 20 wt.%), where M is the sum of the alkaline earth metals Mg, Ca, Sr, and Ba, in the glass. In some examples, the glass can comprise from 2 to 15 wt.% MO. In some examples, the glass can comprise about 2, 3, 4, 5, 6, 7, 8, 9, 10, 11, 12, 13, 14, 15, 16, 17, 18, 19, or 20 wt.% MO.

In some embodiments, the glasses comprise ZnO. In some examples, the glass can comprise 0-10 wt.% ZnO. In some examples, the glass can comprise from 0 to 5 wt.% ZnO. In some examples, the glass can comprise from >0 to 10 wt.%, 3 to 10 wt.%, or 3 to 8 wt.% ZnO. In some examples, the glass can comprise from 0 to 10 wt.%, 0 to 8 wt.%, 0 to 6 wt.%, 0 to 4 wt.%, 0 to 2 wt.%, >0 to 10 wt.%, >0 to 8 wt.%, >0 to 6 wt.%, >0 to 4 wt.%, >0 to 2 wt.%,1 to 10 wt.%, 1 to 8 wt.%, 1 to 6 wt.%, 1 to 4 wt.%, 1 to 2 wt.%, 3 to 8 wt.%, 3 to 6 wt.%, 3 to 10 wt.%, 5 to 8 wt.%, 5 to 10 wt.%, 7 to 10 wt.%, or 8 to 10 wt.% ZnO. In some examples, the glass can comprise about 0, >0, 1, 2, 3, 4, 5, 6, 7, 8, 9, or 10 wt.% ZnO.

Additional components can be incorporated into the glass to provide additional benefits or may be incorporated as contaminants typically found in commercially-prepared glass. For example, additional components can be added as coloring or fining agents (e.g., to facilitate removal of gaseous inclusions from melted batch materials used to produce the glass) and/or for other purposes. In some examples, the glass may comprise one or more compounds useful as ultraviolet radiation absorbers. In some examples, the glass can comprise 3 wt.% or less ZnO, TiO₂, CeO, MnO, Nb₂O₅, MoOs, Ta₂O₅, WO₃, SnO₂, Fe₂O₃, As₂O₃, Sb₂O₃, Cl, Br, or combinations thereof. In some examples, the glass can comprise from 0 to about 3 wt.%, 0 to about 2 wt.%, 0 to about 1 wt.%, 0 to 0.5 wt.%, 0 to 0.1 wt.%, 0 to 0.05 wt.%, or 0 to 0.01 wt.% ZnO, TiO₂, CeO, MnO, NbzOs, MoO₃, Ta₂O₅, WO₃, SnO₂, Fe₂O₃, As₂O₃, Sb₂O₃, Cl, Br, or combinations thereof. The glasses, according to some examples, can also include various contaminants associated with batch materials and/or introduced into the glass by the melting, fining, and/or forming equipment used to produce the glass. For example, in some embodiments, the glass can comprise from 0 to about 3 wt.%, 0 to about 2 wt.%, 0 to about 1 wt.%, 0 to about 0.5 wt.%, 0 to about 0.1 wt.%, 0 to about 0.05 wt.%, or 0 to about 0.01 wt.% SnO₂ or Fe₂O₃, or combinations thereof.

Non-limiting examples of amounts of precursor oxides for forming the embodied glasses are listed in Table 1, along with the properties of the resulting glasses.

**Table 1**

| Oxide (wt%) | 1 | 2 | 3 | 4 | 5 | 6 | 7 |
|---|---|---|---|---|---|---|---|
| SiO₂ | 65.5 | 64.3 | 64.3 | 63.2 | 62.1 | 64.3 | 63.2 |
| B₂O₃ | 0.0 | 0.0 | 0.0 | 0.0 | 0.0 | 0.0 | 0.0 |
| P₂O₅ | 7.4 | 9.1 | 7.3 | 7.1 | 7.0 | 7.3 | 7.1 |
| Al₂O₃ | 0.0 | 0.0 | 0.0 | 0.0 | 0.0 | 0.0 | 0.0 |
| Li₂O | 0.0 | 0.0 | 0.0 | 0.0 | 0.0 | 0.0 | 0.0 |
| Na₂O | 22.5 | 22.1 | 22.1 | 21.7 | 21.3 | 22.1 | 21.7 |
| K₂O | 0.0 | 0.0 | 1.8 | 3.6 | 5.3 | 0.0 | 0.0 |
| MgO | 0.0 | 0.0 | 0.0 | 0.0 | 0.0 | 1.8 | 3.6 |
| CaO | 4.6 | 4.5 | 4.5 | 4.4 | 4.3 | 4.5 | 4.4 |
| ZnO | 0.0 | 0.0 | 0.0 | 0.0 | 0.0 | 0.0 | 0.0 |
| ZrO₂ | 0.0 | 0.0 | 0.0 | 0.0 | 0.0 | 0.0 | 0.0 |
| F⁻ | 0.0 | 0.0 | 0.0 | 0.0 | 0.0 | 0.0 | 0.0 |

The glass compositions disclosed herein can be in any form that is useful for the medical and dental processes disclosed. The compositions are in the form of, fibers.

### Processes for Making Glasses

Glasses having the oxide contents listed in Table 1 can be made via traditional methods. For example, in some examples, the precursor glasses can be formed by thoroughly mixing the requisite batch materials (for example, using a turbular mixer) in order to secure a homogeneous melt, and subsequently placing into silica and/or platinum crucibles. The crucibles can be placed into a furnace and the glass batch melted and maintained at temperatures ranging from 1250-1650°C for times ranging from about 6-16 hours. The melts can thereafter be poured into steel molds to yield glass slabs. Subsequently, those slabs can be transferred immediately to an annealer operating at about 500-650°C, where the glass is held at temperature for about 1 hour and subsequently cooled overnight. In another non-limiting example, precursor glasses are prepared by dry blending the appropriate oxides and mineral sources for a time sufficient to thoroughly mix the ingredients. The glasses are melted in platinum crucibles at temperatures ranging from about 1100°C to about 1650°C and held at temperature for about 16 hours. The resulting glass melts are then poured onto a steel table to cool. The precursor glasses are then annealed at appropriate temperatures.

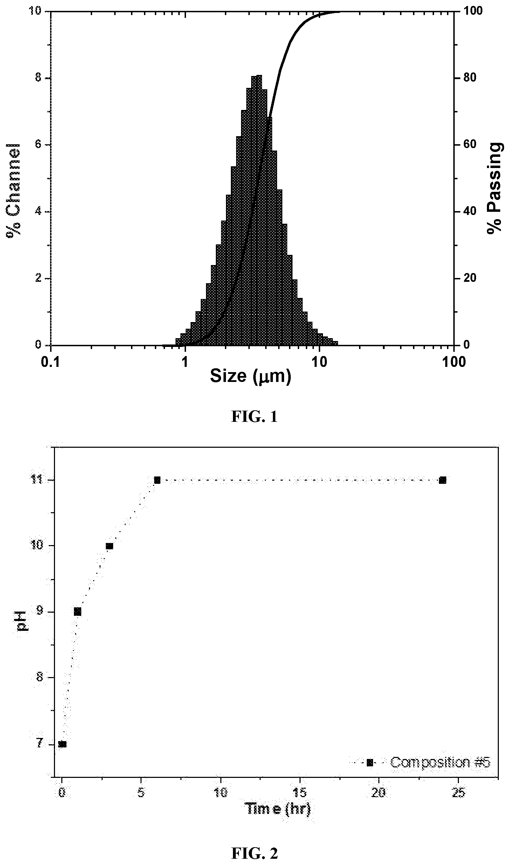

The embodied glass compositions can be ground into fine particles in the range of 1-10 microns (µm) by air jet milling or short fibers. For example, FIG. 1 illustrates a particle size distribution of Example Composition 5 after air jet milling. The particle size can be varied in the range of 1-100 µm using attrition milling or ball milling of glass frits. Furthermore, these glasses can be processed into short fibers using different methods. Short fibers are made by melt spinning or electric spinning. Glasses of desired forms can be used to support cell growth, soft and hard tissue regeneration, stimulation of gene expression or angiogenesis.

Continuous fibers can be easily drawn from the claimed composition using processes known in the art. For example, fibers can be formed using a directly heated (electricity passing directly through) platinum bushing. Glass cullet is loaded into the bushing, heated up until the glass can melt. Temperatures are set to achieve a desired glass viscosity (usually <1000 poise) allowing a drip to form on the orifice in the bushing (Bushing size is selected to create a restriction that influences possible fiber diameter ranges). The drip is pulled by hand to begin forming a fiber. Once a fiber is established it is connected to a rotating pulling/collection drum to continue the pulling process at a consistent speed. Using the drum speed (or revolutions per minute RPM) and glass viscosity the fiber diameter can be manipulated - in general the faster the pull speed, the smaller the fiber diameter. Glass fibers with diameters in the range of 1-100 µm can be drawn continuously from a glass melt. Fibers can also be created using an updraw process. In this process, fibers are pulled from a glass melt surface sitting in a box furnace. By controlling the viscosity of the glass, a quartz rod is used to pull glass from the melt surface to form a fiber. The fiber can be continuously pulled upward to increase the fiber length. The velocity that the rod is pulled up determines the fiber thickness along with the viscosity of the glass.

### Glass Bioactivity

Aspects are related to compositions or matrices containing embodied bioactive glass compositions and the methods of using the matrices to treat medical conditions. The matrices can be a toothpaste, mouthwash, rinse, spray, ointment, salve, cream, bandage, polymer film, oral formulation, pill, capsule, transdermal formulation, and the like. The bioactive glass compositions claimed can be physically or chemically attached to matrices or other matrix components, or simply mixed in. The methods of using the glass-containing matrices to treat a medical condition can be simply like the use of matrix as normally applied.

### E. Coli Applications

Glass particles comprising the compositions disclosed herein may demonstrate strong antimicrobial effectiveness against *E. coli* bacteria. A minimal inhibitory concentration (MIC, corresponding to a 6-log kill rate) as low as 10 mg/mL was determined for Example Composition 5 from Table 1, which is lower than conventional glass particles, such as those with CuNO₃-IOX'd soda lime silicate glass compositions. Table 2 describes the MIC of Example Composition 5 and CuNO₃-IOX'd soda lime silicate glass, both as particles, required to exhibit strong antimicrobial effectiveness against *E. coli* bacteria.

**Table 2**

| Particle | Example Composition 5 | CuNO₃-IOX'd soda lime silicate glass |
|---|---|---|
| MIC (mg/mL) | 10 | 20 |

Testing for the *E. coli* kill rate was determined by first culturing the test bacteria in a suitable medium for 16 to 18 hrs till a saturation point and calculating bacteria concentration by spectrophotometer. Thereafter, the *E. coli* bacteria (107 live *E. coli*) medium is infused with varying concentrations of glass particles. For example, each broth comprises approximately 5 mL of *E. coli* bacteria medium and 0.5, 1, 10, 20, 50, or 200 mg of glass particles per mL of broth, respectively. In addition, a separate broth comprises *E. coli* bacteria medium without glass particles. The inoculation of the bacteria/glass is cultured for 24 hrs and thereafter, 10 µL bacteria/broth is plated and cultured for an additional 16 hrs on Agar plates. After culturing, the *E. coli* colony number for each condition (glass types, concentrations) is counted.

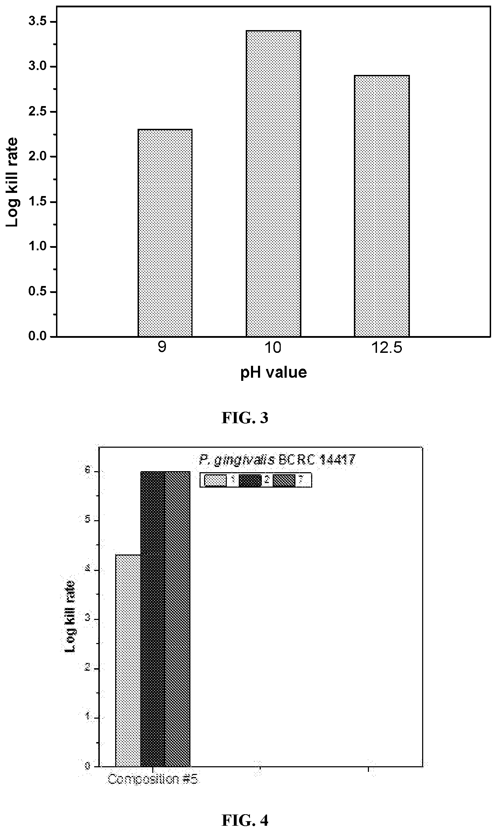

Depending on the Example Glass composition, MIC may vary from 10 to 50 mg/mL. In some examples, antimicrobial effectiveness is associated with alkali and alkali earth ion release from the glass particles. This release may be suggested by pH changes of the broth during testing. For example, FIG. 2 illustrates pH changes of the culture medium as a function of time after soaking with Example Composition 5, according to some embodiments. Rapid pH changes occur from pH=7 to pH=11 within the first 5-7 hrs of culturing and then relatively stabilizes at the upper pH bound thereafter. However, increases of pH alone cannot achieve the required 6-log bacteria kill rates. FIG. 3 illustrates a bar graph plot of kill rate of *E. coli* as a function of pH change of a culture medium, according to some embodiments. None of pH=9, pH=10, or pH=12.5 culture mediums are able to achieve 6-log bacteria kill rates. Although addition of NaOH to the culture medium shows an inhibitory effect on *E. coli* growth, a maximum 3.4-log kill rate was reached at pH=10 (FIG. 3). Further increases to pH do not show advantageous inhibitory effect on the bacteria. Without being bound to theory, it is believed that multiple killing mechanisms, which include pH changes and alkali/alkali earth ion-bacterial interaction, account for the excellent antimicrobial effectiveness from the disclosed compositions.

### P. Gingivalis / S. Mutans Applications

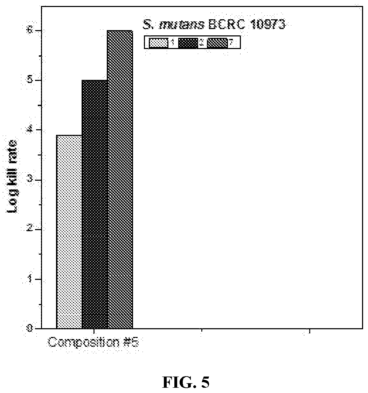

Antibacterial effectiveness may also be demonstrated against other bacteria, such as *P. gingivalis* and *S. mutans,* which are two representative oral bacteria in dental applications. *P. gingivalis* is associated with chronic periodontitis, while *S. mutans* is associated with oral cavity. All disclosed compositions (for example, those in Table 1) demonstrated a 6-log kill rate after 7 days in medium containing either *P. gingivalis* or *S. mutans* bacteria. For example, FIGS. 4 and 5 illustrate bar graph plots of the kill rate of *P. Gingivalis* and *S*. *Mutans,* respectively, after 1, 2 and 7 days of incubation with Example Composition 5, according to some embodiments. Example Composition 5 showed a 4-log kill rate to both bacteria after one day of testing, at least a 5-log kill rate to both bacteria after two days of testing, and a 6-log kill rate to both bacteria after seven days of testing. The antibacterial effectiveness against at least these two representative oral bacteria implies the potential improvement to human oral care using the disclosed glass compositions.

Moreover, because the disclosed compositions (1) show little to no coloring effects of the bacteria-containing medium after soaking for up to 24 hrs or (2) do not contain silver (Ag)- or copper (Cu)-based materials, the compositions are particularly attractive food, water treatment, and paint industry applications.

## Claims

1. An antibacterial composition, comprising:
a silicate-based glass material being a fiber and having a composition of:
60-70 wt.% SiO₂,
0-10 wt.% B₂O₃,
3-18 wt.% P₂O₅,
0-10 wt.% Al₂O₃,
0-5 wt.% Li₂O,
20-30 wt.% Na₂O,
0-15 wt.% K₂O,
0-10 wt.% MgO,
1-15 wt.% CaO,
2-20 wt.% MO, and
20-35 wt.% R₂O,
wherein MO is the sum of MgO, CaO, SrO, and BaO,
wherein R₂O is the sum of Na₂O, K₂O, Li₂O, and Rb₂O, and
wherein the silicate-based glass material is configured to achieve a 6-log kill rate of at least one of *E. coli, P. gingivalis,* or *S. mutans* bacteria.

2. The antibacterial composition of claim 1, wherein the glass material has at least one size dimension in a range of 1-100 µm.

3. The antibacterial composition of claim 1, wherein the glass material has at least one size dimension in a range of 1-10 µm.

4. The antibacterial composition of any one of the preceding claims, wherein the glass material has a minimal inhibitory concentration (MIC) of at most 10 mg/mL in the composition.

5. The antibacterial composition of any one of the preceding claims, wherein the glass material has a minimal inhibitory concentration (MIC) in a range of 10 to 50 mg/mL in the composition.

6. The antibacterial composition of any one of the preceding claims, further comprising:
a culture medium having a pH in a range of 7 to 11.

7. The antibacterial composition of any one of the preceding claims, wherein the antibacterial composition does not comprise silver (Ag)- or copper (Cu)-based materials.

8. The antibacterial composition of any one of the preceding claims, wherein the antibacterial composition is colorless or essentially colorless.

9. The antibacterial composition of any one of the preceding claims, wherein the antibacterial composition comprises at least one of:
5-15 wt.% P₂O₅, 20 -25 wt.% Na₂O, 0-8 wt.% K₂O, 1-10 wt.% CaO, 0-5 wt.% MgO, or >0-8 wt.% ZnO.

10. The antibacterial composition of claim 1, wherein the antibacterial composition comprises:
5-15 wt.% P₂O₅; and 1-10 wt.% CaO.

11. The antibacterial composition of claim 1, wherein the antibacterial composition comprises:
1-10 wt.% CaO; and is essentially free of Al₂O₃.

12. The antibacterial composition of claim 1, wherein the antibacterial composition comprises:
1-10 wt.% CaO; and is essentially free of B₂O₃.

13. The antibacterial composition of any one of the preceding claims, wherein the antibacterial composition further comprises:
0-5 wt.% ZrO₂.

14. The antibacterial composition of any one of the preceding claims, wherein the antibacterial composition is essentially free of or comprises 1 wt.% or less of Li₂O.

15. The antibacterial composition of claim 1, wherein the antibacterial composition is essentially free of or comprises 1 wt.% or less of B₂O₃, Al₂O₃, and Li₂O.

## Patentansprüche

1. Antibakterielle Zusammensetzung, umfassend:
ein Glasmaterial auf Silikatbasis, das eine Faser ist und eine Zusammensetzung aus Folgendem aufweist:
60-70 Gew.-% SiO₂,
0-10 Gew.-% B₂O₃,
3-18 Gew.-% P₂O₅,
0-10 Gew.-% Al₂O₃,
0-5 Gew.-% Li₂O,
20-30 Gew.-% Na₂O,
0-15 Gew.-% K₂O,
0-10 Gew.-% MgO,
1-15 Gew.-% CaO,
2-20 Gew.-% MO und
20-35 Gew.-% R₂O,
wobei MO die Summe aus MgO, CaO, SrO und BaO ist,
wobei R₂O die Summe aus Na₂O, K₂O, Li₂O und Rb₂O ist und
wobei das Glasmaterial auf Silikatbasis dazu konfiguriert ist, eine 6-log-Abtötungsrate von mindestens einem von den Bakterien *E. coli, P. gingivalis* oder *S. mutans* zu erreichen.

2. Antibakterielle Zusammensetzung nach Anspruch 1, wobei das Glasmaterial mindestens eine Größenabmessung in einem Bereich von 1-100 µm aufweist.

3. Antibakterielle Zusammensetzung nach Anspruch 1, wobei das Glasmaterial mindestens eine Größenabmessung in einem Bereich von 1-10 µm aufweist.

4. Antibakterielle Zusammensetzung nach einem der vorhergehenden Ansprüche, wobei das Glasmaterial eine minimale Hemmkonzentration (MIC) von höchstens 10 mg/ml in der Zusammensetzung aufweist.

5. Antibakterielle Zusammensetzung nach einem der vorhergehenden Ansprüche, wobei das Glasmaterial eine minimale Hemmkonzentration (MIC) in einem Bereich von 10 bis 50 mg/ml in der Zusammensetzung aufweist.

6. Antibakterielle Zusammensetzung nach einem der vorhergehenden Ansprüche, ferner umfassend:
ein Kulturmedium mit einem pH-Wert im Bereich von 7 bis 11.

7. Antibakterielle Zusammensetzung nach einem der vorhergehenden Ansprüche, wobei die antibakterielle Zusammensetzung keine Materialien auf Basis von Silber (Ag) oder Kupfer (Cu) umfasst.

8. Antibakterielle Zusammensetzung nach einem der vorhergehenden Ansprüche, wobei die antibakterielle Zusammensetzung farblos oder im Wesentlichen farblos ist.

9. Antibakterielle Zusammensetzung nach einem der vorhergehenden Ansprüche, wobei die antibakterielle Zusammensetzung mindestens eines von Folgenden umfasst:
5-15 Gew.-% P₂O₅, 20-25 Gew.-% Na₂O, 0-8 Gew.-% K₂O, 1-10 Gew.-% CaO, 0-5 Gew.-% MgO oder >0-8 Gew.-% ZnO.

10. Antibakterielle Zusammensetzung nach Anspruch 1, wobei die antibakterielle Zusammensetzung Folgendes umfasst:
5-15 Gew.-% P₂O₅; und 1-10 Gew.-% CaO.

11. Antibakterielle Zusammensetzung nach Anspruch 1, wobei die antibakterielle Zusammensetzung Folgendes umfasst:
1-10 Gew.-% CaO; und im Wesentlichen frei von Al₂O₃ ist.

12. Antibakterielle Zusammensetzung nach Anspruch 1, wobei die antibakterielle Zusammensetzung Folgendes umfasst:
1-10 Gew.-% CaO; und im Wesentlichen frei von B₂O₃ ist.

13. Antibakterielle Zusammensetzung nach einem der vorhergehenden Ansprüche, wobei die antibakterielle Zusammensetzung ferner Folgendes umfasst:
0-5 Gew.-% ZrO₂.

14. Antibakterielle Zusammensetzung nach einem der vorhergehenden Ansprüche, wobei die antibakterielle Zusammensetzung im Wesentlichen frei von Li₂O ist oder 1 Gew.-% oder weniger davon umfasst.

15. Antibakterielle Zusammensetzung nach Anspruch 1, wobei die antibakterielle Zusammensetzung im Wesentlichen frei von B₂O₃, Al₂O₃ und Li₂O ist oder 1 Gew.-% oder weniger davon umfasst.

## Revendications

1. Composition antibactérienne, comprenant :
un matériau en verre à base de silicate étant une fibre et comportant une composition de : 60 à 70 % en poids de SiO₂,
0 à 10 % en poids de B₂O₃,
3 à 18 % en poids de P₂O₅,
0 à 10 % en poids d' Al₂O₃,
0 à 5 % en poids de Li₂O,
20 à 30 % en poids de Na₂O,
0 à 15 % en poids de K₂O,
0 à 10 % en poids de MgO,
1 à 15 % en poids de CaO,
2 à 20 % en poids de MO, et
20 à 35 % en poids de R₂O,
MO représentant la somme de MgO, CaO, SrO et BaO,
R₂O représentant la somme de Na₂O, K₂O, Li₂O et Rb₂O, et
ledit matériau en verre à base de silicate étant conçu pour atteindre un taux de destruction de 6-log d'au moins une bactérie parmi les bactéries *E. coli, P. gingivalis* ou *S. mutans.*

2. Composition antibactérienne selon la revendication 1, ledit matériau en verre comportant au moins une dimension de taille dans la plage de 1 à 100 µm.

3. Composition antibactérienne selon la revendication 1, ledit matériau en verre comportant au moins une dimension de taille dans la plage de 1 à 10 µm.

4. Composition antibactérienne selon l'une quelconque des revendications précédentes, ledit matériau en verre comportant une concentration inhibitrice minimale (MIC) d'au plus 10 mg/ml dans la composition.

5. Composition antibactérienne selon l'une quelconque des revendications précédentes, ledit matériau en verre comportant une concentration inhibitrice minimale (MIC) dans la plage de 10 à 50 mg/ml dans la composition.

6. Composition antibactérienne selon l'une quelconque des revendications précédentes, comprenant en outre :
un milieu de culture comportant un pH dans la plage de 7 à 11.

7. Composition antibactérienne selon l'une quelconque des revendications précédentes, ladite composition antibactérienne ne comprenant pas de matériaux à base d'argent (Ag) ou de cuivre (Cu).

8. Composition antibactérienne selon l'une quelconque des revendications précédentes, ladite composition antibactérienne étant incolore ou sensiblement incolore.

9. Composition antibactérienne selon l'une quelconque des revendications précédentes, ladite composition antibactérienne comprenant au moins un élément parmi : 5 à 15 % en poids de P₂O₅, 20 à 25 % en poids de Na₂O, 0 à 8 % en poids de K₂O, 1 à 10 % en poids de CaO, 0 à 5 % en poids de MgO, ou > 0 à 8 % en poids de ZnO.

10. Composition antibactérienne selon la revendication 1, ladite composition antibactérienne comprenant :
5 à 15 % en poids de P₂O₅ ; et 1 à 10 % en poids de CaO.

11. Composition antibactérienne selon la revendication 1, ladite composition antibactérienne comprenant :
1 à 10 % en poids de CaO ; et étant sensiblement exempte d' Al₂O₃.

12. Composition antibactérienne selon la revendication 1, ladite composition antibactérienne comprenant :
1 à 10 % en poids de CaO ; et étant sensiblement exempte B₂O₃.

13. Composition antibactérienne selon l'une quelconque des revendications précédentes, ladite composition antibactérienne comprenant en outre :
0 à 5 % en poids de ZrO₂.

14. Composition antibactérienne selon l'une quelconque des revendications précédentes, ladite composition antibactérienne étant sensiblement exempte de ou comprenant 1 % en poids ou moins de Li₂O.

15. Composition antibactérienne selon l'une quelconque des revendications précédentes, ladite composition antibactérienne étant sensiblement exempte de ou comprenant 1 % en poids ou moins de B₂O₃, d' Al₂O₃ et de Li₂O.
